(19) Europäisches Patentamt
European Patent Office
Office européen des brevets

(11) EP 1 111 995 B1

(12) EUROPEAN PATENT SPECIFICATION

(45) Date of publication and mention of the grant of the patent:
**09.02.2005 Bulletin 2005/06**

(21) Application number: **99949638.3**

(22) Date of filing: **10.09.1999**

(51) Int Cl.[7]: **A01N 47/44**
// (A01N47/44, 61:00, 59:16, 59:12, 47:44, 37:40, 33:12), A01N31:02

(86) International application number:
**PCT/US1999/020976**

(87) International publication number:
**WO 2000/015036 (23.03.2000 Gazette 2000/12)**

# (54) TOPICAL DERMAL ANTIMICROBIAL COMPOSITIONS

TOPISCH-DERMALE ANTIMIKROBIELLE ZUSAMMENSETZUNGEN

COMPOSITIONS ANTIMICROBIENNES DERMIQUES TOPIQUES

(84) Designated Contracting States:
**AT BE CH CY DE DK ES FI FR GB GR IE IT LI LU MC NL PT SE**

(30) Priority: **11.09.1998 US 99925 P**
**15.01.1999 US 116013 P**

(43) Date of publication of application:
**04.07.2001 Bulletin 2001/27**

(73) Proprietor: **Surfacine Development Company, LLC**
**Tewksbury, MA 01876-1274 (US)**

(72) Inventors:
- **SAWAN, Samuel, P.**
  **Tyngsboro, MA 01879 (US)**
- **SUBRAMANYAM, Sundar**
  **Stoneham, MA 02180 (US)**
- **YURKOVETSKIY, Alexander**
  **Acton, MA 01720 (US)**
- **MANIVANNAN, Gurusamy**
  **N. Chelmsford, MA 01863 (US)**
- **GOLDBLATT, Michael**
  **McLean, VA 22101-2203 (US)**

(74) Representative: **Wallace, Sheila Jane et al**
**Lloyd Wise**
**Commonwealth House,**
**1-19 New Oxford Street**
**London WC1A 1LW (GB)**

(56) References cited:
**EP-A- 0 450 117**
**WO-A-98/18330**
**WO-A-99/40791**
**DE-A- 19 646 759**
**US-A- 4 478 821**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates generally to topical antimicrobial compositions having initial and residual disinfectant activity. The invention also relates more specifically to topical dermal antiseptic compositions containing a self-preserving antimicrobial polymer that exhibit sanitizing properties when applied on skin, and form microbial barrier films in-situ that are moisture and sweat resistant, and provide "persistence" or extended duration residual antimicrobial efficacy in water contacting environments, and deodorizing action that is moisture and sweat resistant.

### Background of the Invention

The constant threat of bacterial contamination and the associated repercussions on health have made antiseptic products such as antimicrobial creams, lotions, sprays, gels, foams and deodorants a ubiquitous part of personal hygiene. Antiseptic sanitizers are especially important in susceptible environments such as hospitals, healthcare facilities and food service areas in residential kitchens and in restaurants. Common antiseptic products that contain ethanol; isopropanol, triclosan, etc. provide sanitizing efficacy upon application. Such efficacy, however, is short lived, since such products are incapable of providing long lasting protection in terms of persistent residual antimicrobial action. This often results in recontamination of surfaces, requiring frequent reapplication of antiseptic. Relatively high concentrations of the active agent must be incorporated in these formulations in order to obtain broad-spectrum activity. Such high concentrations or repeated reapplication often have undesirable side effects, particularly when the agent is applied to skin. These side effects can include, for example, skin irritation and dermal sensitization. These compounds can also contaminate food products and can be responsible for unpleasant tastes or odors and can be potentially harmful to consumers if ingested. Furthermore, presently available antiseptic formulations make it difficult, if not impossible, for employers to monitor individual compliance with sanitary procedures. Food service or healthcare workers who fail to adhere to proper hygiene and sanitary procedures can potentially transmit pathogenic bacteria to unsuspecting consumers or patients.

Dermal antiseptic formulations are generally categorized as surgical scrubs, preoperative skin preparations, healthcare personnel handwashes, food handler handwashes and general population handwashes. This categorization is based on efficacy of such products against pathogenic microorganisms relevant to the area of use. They contain either a single antimicrobial agent or a mixture of more than one agent that are considered "active ingredients". The type of active ingredients used in an antiseptic formulation is dependent on the category of its use. Formulations used as surgical scrubs and pre-operative skin preparations typically contain alcohol (ethanol, n-propanol or isopropanol), chlorhexidine gluconate, iodine, or povidone-iodine complex. Such agents have a number of limitations, such as skin dehydration causing dryness (in the case of alcohols), skin irritation and sensitization (in the case of chlorhexidine and iodine), and skin discoloration (in the case of iodine and its complexes). Alcohol and iodine based antiseptics do not exhibit residual antimicrobial activity or "persistence" that is a prerequisite characteristic for products in this category, due to their volatility. They must therefore be either be formulated with emollients to retard evaporation from the skin (for alcohols) or complexed to control their release (povidone-iodine). Furthermore, active ingredients for formulations used in other categories such as triclosan, triclorocarban and *para*-chloro-*meta*-xylenol (PCMX) are restricted by regulations for use as surgical scrubs.

Alcohol-based antiseptics for use in dermal applications such as surgical scrubs, preoperative skin preparations and antiseptic handwashes are well known and widely used because of their high effectiveness and the rapidity with which they kill microorganisms, as well as their non cytotoxicity. Alcohol containing formulations, containing 60-95% by volume of ethanol or isopropanol, are often used as surgical scrubs, in preoperative skin preparations, as healthcare personnel handwashes and antiseptic handwashes to disinfect hands, and for localized skin disinfection at the site of an invasive medical procedure. The efficacy of such compositions is short term, due to rapid evaporation of the alcohol, which is the antimicrobially active ingredient. Other limitations resulting from the use of such formulations include skin dryness and difficulty in application due to their low viscosity and watery nature. Their use in applications requiring sustained antimicrobial efficacy (persistence), such as surgical scrubs, is therefore limited by their high vapor pressure. (which causes rapid evaporation upon application). Thus, when applied to skin, the rapid decrease in alcohol concentration limits the agent's contact time with microbes, especially bacteria, due to evaporative loss. U.S. Patent No. 5,288,486 discloses a method to decrease the evaporation rate of alcohol by addition of an alcohol soluble viscosifying agent to prolong its activity. Such compositions, however, are not capable of providing a film on skin that can continue to exhibit antimicrobial activity over extended periods, especially after contacting water or aqueous solutions. These compositions do not provide residual barrier properties precluding bacterial penetration of the barrier and subsequent contamination and proliferation on the underlying skin

international Application No. WO-A-98/18330 discloses an antimicrobial material which can be used to form on the surface of a substrate a non-leaching antimicrobial coating or layer. The coating comprises an organic matrix having biocidal metallic materials associated with it.

US Patent No. 4,478,821 discloses the use of polyhexamethylene biguanide hydrochloride in a dermatologically acceptable carrier in order to inhibit body odour.

EP-A-0 450 117 discloses a lactate-free pure Ringer's solution which additionally contains 0.1% of a concentrate comprising polyhexamethylene biguanidine hydrochloride and polyethylene glycol. This can be used for local bacterial decontamination of wounds.

DE-A-196 46 759 discloses compositions for disinfecting the skin which contain optical brightener which exhibits intense fluorescence. The compositions permit simple monitoring of treated skin for ensuring complete disinfection.

There are other examples involving utilization of compositions that provide an antimicrobial barrier film on skin. Such compositions involve dispersing a water soluble, low molecular weight antimicrobial agent in a film-forming polymer matrix. U.S. Patent No. 5,417,968 discloses an antimicrobial barrier composition wherein an antimicrobial compound is mixed with a film-forming polymer that can form a barrier film on skin, and allows for elution of the antimicrobial compound to impart antimicrobial property to the film. U.S. Patent No. 4,374,126 discloses a skin adherent polymeric film into which an antimicrobial compound is impregnated so as to provide antimicrobial action via diffusion. Antimicrobial action of resultant films is effected by elution of the antimicrobial agent from the matrix via dissolution or diffusion. Such barrier films do not provide the rapid disinfection that is required of a surgical scrub, nor are they capable of providing extended antimicrobial efficacy after contact with water or aqueous solutions. Antimicrobial compositions disclosed in the prior art are therefore incapable of providing rapid antiseptic action and persistent antimicrobial efficacy, especially upon contact with water or aqueous solutions, for example, after a handwash. Such formulations would have to be reapplied to prevent subsequent microbial contamination. This limitation requires frequent use of the antiseptic, especially in hospital, healthcare and food handling areas where bacterial contamination can result in serious consequences. Furthermore, because these formulations elute when exposed to aqueous solutions (*e.g*. sweat), they can penetrate the skin barrier, rendering them potentially skin irritating and sensitizing with prolonged or continuous use. Such compositions do not provide microbial barrier properties or antimicrobial polymers that continue to exhibit antimicrobial efficacy on skin after contacting water as when handwashing. In regulated environments such as the healthcare and food industries, there is also a need to develop means to monitor employees' compliance with hygiene and sanitary procedures involving the use of dermal antiseptics and hand sanitizers.

Thus, there is a need to develop new, non-irritating disinfecting formulations that can provide fast-acting, broad-spectrum, persistent antimicrobial activity on surfaces, e.g. skin, without reapplication, even after contacting water. There is a need for antiseptic formulations that are self-preserving, that is, for formulations that do not require the addition of a soluble, low molecular weight antimicrobial agent to inhibit bacterial growth on a polymeric film formed in-situ on a surface and resistant to water.

There is a need for dermal antiseptic formulations exhibiting persistent efficacy and microbial barrier properties using low levels of the antimicrobial agent, thus avoiding skin irritation or sensitization for the user. Additionally, there is a need for dermal antiseptic formulations whose presence on skin can be readily determined.

There is a need for efficient deodorants that are moisture and sweat resistant. Antimicrobial deodorant compositions reported in the prior art are either effective for short duration due to volatility of the active agent (*e.g*., alcohol) or are dissolved in sweat and dispersed, thereby rendering them effective only for short periods. Such deodorants must therefore be formulated in combination with an antiperspirant agent for extended duration efficacy. Such agents may cause skin dryness, irritation and sensitization. It is therefore desirable to have deodorants that are sweat and moisture resistant that provide extended duration deodorizing activity without the use of an antiperspirant agent.

## Summary of the Invention

It is an object of the present invention to provide a non-eluting topical antimicrobial composition which is capable of (i) providing immediate broad-spectrum antimicrobial disinfection and (ii) providing sustained or residual antimicrobial disinfecting action for extended periods after application, even after being contacted by water or other liquids. An additional object of the invention is to provide a composition that can bind non-leachably to a surface. A further object of the invention is to provide an antimicrobial material that does not release biocidal amounts of leachables into a contacting solution. Another object of the invention is to provide a substantially water-insoluble, self-preserving microbial barrier film that imparts "persistence" (residual antimicrobial action) for extended periods after application. An additional object of the invention is to provide deodorizing action of extended duration on skin even after exposure to moisture and sweat. Another object of the invention is to provide a topical antimicrobial composition which comprises an optical reporter, e.g., a fluorophore or an optical brightening agent that enables detection of the presence of the topical antimicrobial composition on skin surfaces by suitable detection devices such as irradiation by an ultraviolet, fluorescent, infrared,. or visible light source.

A further object of the present invention is to provide methods for detecting the presence of antimicrobial compositions on skin surfaces by providing a topical antimicrobial composition that contains an optical reporter. An

additional object of the present invention is to provide methods for monitoring a subject's compliance with sterile or sanitary procedures by providing an antimicrobial composition that contains a marker and subsequently exposing the subject to a detector capable of detecting the presence of the marker on the subject in order to determine whether the subject applied the composition.

Another object of the present invention is to provide a non-eluting, self-preserving polymeric antimicrobial material that (i) forms a microbial barrier in-situ upon application to skin and (ii) is capable of inhibiting microbial growth and preventing microbes from growing through the barrier over extended periods to the surface of underlying skin. It is an object of the invention to render the self-preserving polymeric material substantially water-insoluble so as to make it non-eluting; that is, it does not dissolve, elute or leach into contacting aqueous solutions at bactericidal levels. This is accomplished by making it conducive to spontaneously associating with skin and bonding to it via electrostatic, ionic or covalent bonding.

It is also an object of the invention to render the self-preserving polymer further water-insoluble by reacting it with a hydrophobic organic compound. Such a modification renders the polymeric antimicrobial material substantially water-insoluble. thereby enabling it to efficiently associate with skin upon application.

Another object of the invention is to react the antimicrobial polymeric material with a covalent coupling agent so that the resulting adduct is capable of forming covalent chemical bonds with functionalities such as amino, sulfhydryl, or carboxylic acid groups. Such covalent bonding enables retention of the antimicrobial polymer upon its application to an appropriate surface. Thus, the antimicrobial polymer provides a non-leachable, non-eluting microbial barrier that is capable of rapid sanitation and persistent antimicrobial activity that is substantially undiminished even upon contacting water.

An additional object of the present invention is to provide a topical antimicrobial composition comprising a second antimicrobial component non-leachably dispersed in the self-preserving barrier-forming antimicrobial polymeric material such that the antimicrobial component is capable of enhancing the persistent antimicrobial efficacy of the latter by killing microorganisms on contact without leaching from the composition into the surrounding environment at levels toxic to microorganisms. Such antimicrobial compositions are capable of providing residual antimicrobial activity on dermal surfaces even after repeated exposure to aqueous solutions and thus are particularly, useful as dermal antiseptics and hand sanitizers.

Thus, the present invention discloses compositions for a dermal antiseptic that exhibits rapid antimicrobial action upon application to skin, and provides residual antimicrobial action (persistence) over extended time periods even upon contact with water. The scope of the present invention is not limited to alcohol-containing skin antiseptics, and can be extended to non-alcohol-containing formulations that contain antimicrobially active materials such as chlorhexidine, iodine, povidone-iodine complexes, triclosan, triclorocarban, para-chloro-meta-xylenol, etc., in aqueous or organic solvents to provide similar benefits.

The biocide preferably is non-leachably attached to, complexed or associated with or dispersed within the film, but is capable of being preferentially transferred directly from the polymeric film to the contacting microorganism due to a higher affinity of the biocide for proteins within the microorganism.

In one aspect, the present invention relates to the use of a water-insoluble adduct of a polycationic organic polymer with a hydrophobic agent in an antimicrobial composition without an antimicrobial metal. There is also provided a method for detecting the presence of an antimicrobial composition on a surface, the method comprising the steps of:

(i) providing on the surface the dermal antimicrobial composition; and

(ii) exposing the surface to a detector capable of detecting the presence of the marker on the surface.

The polymeric material preferably comprises a polymer, copolymer or adduct which contains segments that, when the polymer forms a film on a surface, are capable of engaging microorganisms that come into contact with it.

The organic material must possess two important properties: it must be capable of reversibly binding or complexing with the biocide, and must be capable of insinuating the biocide into the cell membrane of a microorganism in contact with it. The organic material preferably is capable of disrupting or interacting with the cell membrane surrounding the microorganism. Preferred organic materials are those which can be applied on a surface as substantially water-insoluble films and which bind the biocide in such a manner as to permit transfer of the biocide into the microorganism without releasing the biocide (at biocidal levels) into the surrounding environment, e.g., into the air or into any liquid in contact with the coated surface. Preferred organic materials are polycationic polymeric antimicrobial materials such as biguanide polymers. Especially preferred biguanide polymers include poly(hexamethylenebiguanide), poly(hexamethylenebiguanide) hydrochloride, or derivatives thereof.

The antimicrobial materials of the present invention are, therefore, molecularly designed to enable a matrix-bound biocide to retain high antimicrobial activity without elution of any compounds into contacting solutions, carriers or other materials. The antimicrobial's activity .. stems from the sustained, cooperative biocidal action of its components.

Selective transfer of one component from within the matrix directly to the microorganism upon contact is achieved via a "handoff" mechanism upon engagement and penetration of the microorganism's cell membrane by the organic material. The antimicrobial material, therefore, maintains long term efficacy without releasing toxic elutables into the surrounding environment. Components that can be used in the present invention to provide cooperative biocidal action are non metallic biocides.

The invention comprises topical compositions for immediate sanitation of a surface, such as skin, providing long-term residual antimicrobial efficacy or "persistence" over extended duration, even after being contacted with water under conditions simulating a hand rinse. In one embodiment, the formulation is a topical composition comprising a solution, dispersion or suspension of the organic polymeric antimicrobial material and the biocidal material in a suitable carrier. The composition need not be a homogeneous solution. If desired, stabilizing agents such as suspending agents or surface active agents may be included. The topical composition may also include an optical reporter, e.g., a fluorophore or an optical brightening agent that enables detection of the presence of the topical composition (e.g. the microbial barrier formed on skin) by use of suitable detection devices, such as irradiation by an ultraviolet, fluorescent, infrared, or visible light source.

There is described a method to enhance the activity of dermal antiseptic formulations that overcome the limitations of formulations known in the prior art and can be used in all of the above-mentioned categories of dermal antiseptics and disinfectants, since they confer on such formulations antimicrobial persistence that remains unaffected even after loss of active ingredients in the antiseptic by evaporation or dissolution by water contact. The antimicrobial materials of the present invention may be used to enhance the efficacy of commercial biocidal compositions containing active agents such as alcohol (ethanol and n-isopropanol), chlorhexidine (Hibistat™ and Hibiclens™) or povidone-iodide complex (Betadine™), and enable such formulations to exhibit persistent antimicrobial efficacy even upon being contacted with watfr. Furthermore, the compositions of the present invention provide such antimicrobial activity without producing skin irritation or cytotoxicity.

The limitations in the prior known methods and compositions are overcome by the present invention which relates to the addition of a self-preserving film-forming polymeric antimicrobial agent that enhances the efficacy of alcohol-containing formulations and provides residual antimicrobial efficacy or persistence after alcohol- evaporation, thereby allowing more efficient use of alcohols as skin disinfectants or antiseptics for applications such as surgical scrubs and pre-operative skin preparations where persistence is a necessary attribute. Additionally, the present invention relates to the in-situ formation of a microbial barrier or film on skin upon its application. This microbial barrier or film is self-preserving; it kills contacting microorganisms and prevents them from growing through or penetrating the barrier to underlying skin.

Thus, there are described methods for extending the duration of efficacy of a dermal antiseptic formulation by providing a dermal antiseptic formulation and mixing in a polycationic antimicrobial material, such that the antimicrobial material is capable of forming a self-preserving antimicrobial barrier upon application of the formulation to skin, wherein the barrier inhibits microorganism growth, thereby enhancing the antimicrobial efficacy of the antiseptic formulation by imparting residual antimicrobial activity that is persistent.

In another aspect, the present invention relates to compositions comprising a dermal antiseptic formulation and an organic, polycationic, antimicrobial polymer that binds to skin upon application. In one embodiment, the formulation spontaneously binds to skin upon application, forming a self-preserving antimicrobial barrier that provides persistent antimicrobial activity.

These and other objects, features and advantages of the present invention will be better understood from the following description when read in conjunction with the accompanying drawings and examples.

Brief Description of the Drawings

Figure 1A is a schematic graphic illustration of the polymer/biocide complex of the present invention, forming a film on the surface;

Figure 1B is a schematic graphic illustration of the contact-killing ability of the film-forming matrix/biocide complex of the present invention upon contact of the film with microorganisms, wherein the polymer chains engage and disrupt the microorganism cell membrane; and

Figure 1C is a schematic graphic illustration of the penetration of the cell membrane and transfer of the biocide from the network to proteins in the microorganism, causing cell death.

Figure 2 is a bar graph of the number of colony forming units elutable from a hand after treatment with a surgical

scrub protocol, relative to a control baseline determination. The surgical scrub comprises an adduct of polyhexamethylenebiguanide hydrochloride with methylene-bis-N,N-diglycidylaniiiine. The scale is logarithmic, and the error bars indicate the 95% confidence interval.

Figure 3 is a bar graph of the number of colony forming units elutable from a hand after treatment with a surgical scrub protocol, relative to a control baseline determination. The surgical scrub comprises silver complexed to an adduct of polyhexarnethylenebiguanide hydrochloride with methylene-bis-N,N-diglycidylaniline. The scale is logarithmic, and the error bars indicate the 95% confidence interval.

Detailed Description

The topical antimicrobial compositions of the present invention can be applied directly to the skin's surface to disinfect the area of application upon contact. The antimicrobial compositions also provide residual activity to kill microorganisms contacting the area of application subsequent to the initial treatment.

The term "microorganism" as used herein includes pathogenic organisms and infective agents, including bacteria, viruses, blue-green algae, fungi, yeast, mycoplasmids, protozoa, parasites and algae.

The term "biocidal" as used herein means bactericidal or bacteriostatic. The term "bactericidal" as used herein means the killing of microorganisms. The term "bacteriostatic" as used herein means inhibiting the growth of microorganisms, which can be reversible under certain conditions.

As used herein, the terms "non-eluting", "non-leachable" and "substantially non-leachable" mean that bioactive components in the disinfecting compositions do not dissolve, elute, leach or otherwise provide species into a liquid environment in contact with the compositions at levels that would result in solution disinfection, that is, in antimicrobially effective amounts. Preferably, this threshold is below the minimum solution inhibitory concentrations (MIC) of such components to cause the contacting solution to be biocidal.

As used herein, the terms, "sanitizer" and "antiseptic" refer to those mixtures that are applied to skin for the purpose of killing bacteria and microorganisms on the skin. Such mixtures may be used as surgical scrub hand washes, patient pre-operative skin preparations, and as healthcare personnel, food handler and general population hand washes. Other uses will become apparent to those skilled in the art and are intended to be within the scope of this invention.

The phrase "self-preserving antimicrobial barrier or film" as used herein refers to any antimicrobial polymeric compound that is capable of forming a barrier or film on the surface of a substrate such as skin, and inhibits the proliferation of microorganisms on said film, and prevents them from growing through to underlying skin. The phrase "residual antimicrobial activity" as used herein refers to the activity of any chemical compound that is capable of forming a residue on a substrate surface and, upon its application, is capable of providing either bacteriostatic or bactericidal activity. When present in a dermal antiseptic or disinfectant formulation containing other active antimicrobial agents, the residue obtained from such agent is capable of sanitizing (bactericidal action) or acting as a preservative to prevent organism growth (bacteriostatic action). The term "persistence" as used herein refers to the ability of an antimicrobial material to inhibit bacterial regrowth on skin for an extended period of time after the initial antiseptic action caused by application of the antimicrobial formulation.

Organic materials useful in the present invention comprise antimicrobial materials which are capable of: (1) adhering to and/or forming a layer or coating on a surface such as skin, (2) reversibly binding to or complexing with a biocide to prevent its elution or dissolution, and (3) insinuating the biocide into the cell membrane of contacting microorganisms. A preferred class of materials are those having the aforementioned properties, which are capable of being immobilized on a surface and which preferentially bind to biocidal materials in such a manner so as to permit release of the biocide to the microorganism, but not to the contacting environment. More preferred is the class of organic materials that can attach to a surface by forming covalent chemical bonds with reactive moieties such as amino or carboxylic acid groups. Most preferred is the class of organic materials having antimicrobial properties: materials that, when applied as a coating, can dissolve into, adhere to, disrupt or penetrate the lipid bilayer membrane of a microorganism in contact with the barrier film. In a preferred embodiment, the organic material is a polymer containing segments which, when the polymer forms a coating on a surface, are capable of engaging microorganisms which come into contact with the coating. By "engaging" it is meant that the coating can attach and temporarily immobilize a microorganism in contact with it. The barrier film can dissolve into, or adhere to, and penetrate at least the outer portion of the lipid bilayer membrane of a microorganism. For this purpose, surface active agents, such as cationic compounds, polycationic compounds, anionic compounds, polyanionic compounds, non-ionic compounds, polyanionic compounds or zwitterionic compounds may be used. These compounds include, for example, biguanide polymers, or polymers having side chains containing biguanide moieties or other cationic functional groups, such as benzalkonium groups or quartemium groups (e.g., quarternary amine groups). The polymer backbone may be any polymer capable of forming a coating on a substrate. It is understood that the term "polymer" as used herein includes any organic material comprising three or more repeating units, and includes oligomers, polymers, copolymers, terpolymers, etc. The polymer

backbone may be a polysilane or polyethylene polymer, for example. Organic materials which currently are most preferred for use in the invention are polymeric biguanide compounds. When applied to a substrate, these polymers form a barrier film that can engage and disrupt a microorganism as shown in Figure 1.

Polymeric materials useful in the present invention include polymers containing benzalkoniumchloride or its derivatives, α-4-[1-tris(2-hydroxyethyl) ammonium-2-butenyl] poly[1-dimethylammonium-2-butenyl]-ω -tris(2-hydroxyethyl) ammonium chloride. Preferred polymeric compounds include polymeric biguanides and their salts of the general formula:

$$\begin{array}{c} Y_1\text{-[-NH-}\underset{\underset{\displaystyle Z^-}{\displaystyle NH^+}}{\underset{\|}{C}}\text{-NH-}\underset{\underset{\displaystyle Z^-}{\displaystyle NH^+}}{\underset{\|}{C}}\text{-NH-X-]}_n\text{-}Y_2 \end{array}$$

or their water soluble salts, where X is any aliphatic, cycloaliphatic, aromatic, substituted aliphatic, substituted aromatic, heteroaliphatic, heterocyclic, or heteroaromatic compound, or a mixture of any of these, and $Y_1$ and $Y_2$ are any aliphatic, cycloaliphatic, aromatic, substituted aliphatic, substituted aromatic, heteroaliphatic, heterocyclic, or heteroaromatic compound, or a mixture of any of these, where n is an integer equal to or greater than 1, and wherein Z is an anion such as $Cl^-$ or $OH^-$. In a preferred embodiment, the polymeric material is capable of adsorbing to a surface via electrostatic interaction, ionic interaction, or "hydrophobic forces". In another preferred embodiment, the polymeric material can bond covalently with a surface. Currently, the most preferred polymeric compound is polyhexamethylenebiguanide hydrochloride (available from Avecia, Inc. of Wilmington, DE as a 20% aqueous solution under the trade name COSMOCIL-CQ™). Similarly preferred polymeric compounds include poly(hexamethylenebiguanide) hydrochloride, poly (hexamethylenebiguanide) gluconate, poly(hexamethylenebiguanide) stearate, or poly(hexamethylenebiguanide) derivatives.

In another preferred embodiment, the organic polymeric material may be further reacted with a substantially water-insoluble organic compound or "hydrophobic agent" to form a substantially water-insoluble adduct that is capable of forming a barrier or film *in situ* upon application to skin that is impervious to contact with water or aqueous solutions. As used herein, "substantially water-insoluble" means that bioactive components in the disinfecting compositions do not dissolve, elute, leach or otherwise provide species into a liquid environment in contact with the compositions at levels that would result in solution disinfection, that is, in antimicrobially effective amounts. Preferably, this threshold is below the minimum solution inhibitory concentrations (MIC) of such components to cause the contacting solution to be biocidal. This adduct, when added to a dermal antiseptic formulation or other carrier, confers on it a residual antimicrobial activity or persistence for extended periods of time in water-contacting (aqueous) environments. In a preferred embodiment, the organic material is a polymeric polycationic polymer, which is chemically reacted with a hydrophobic agent to form an adduct. The adduct that includes the hydrophobic agent exhibits greater water-insolubility, thus adhering more strongly to a surface such as skin than does the polycationic polymer alone. Hydrophobic agents which can be used in the present invention are organic compounds which are substantially water-insoluble and which can react with the polycationic material to form an adduct. Suitable hydrophobic agents include, for example, organic compounds containing a multifunctional groups such as a carbodiimide, isocyanate, isothiocyanate, succimidyl ester, epoxide, carboxylic acid, acid chloride, acid halide, acid anhydride, succimidyl ether, aldehyde, ketone, alkyl methane sulfonate, alkyl trifluoromethane sulfonate, alkyl paratoluene methanesulfonate, alkyl halide and organic multifunctional epoxide. In a currently preferred embodiment, a polyhexamethylene biguanide polymer is reacted with an epoxide, such as methylene-bis-N,N-diglycidylaniline, bisphenol-A-epichlorohydrin, or N,N-diglycidyl-4-glycidyloxyaniline. The degree of hydrophobicity of the resulting adduct can be adjusted by choice of the hydiophobic agent. The organic material can be polymeric or non-polymeric, and the resulting adduct may be capable of forming a coherent film.

In another preferred embodiment, the organic polymeric material comprises a chemical group that is capable of forming a covalent bond with a functional group on a substrate. Suitable functional groups on the substrate might include, for example, an amino group, a carboxylic acid group, a hydroxyl group, or a sulfhdryl group. Such a functional group could be found, for example, on a proteinaceous substrate or a substrate comprising a peptide. Appropriate substrates therefore include, for example, proteins, such as collagen, or living tissue, such as skin. In one embodiment, the substrate comprises a reactive chemical group such as a carbodiimide, isocyanate, isothiocyanate, succimidyl ester, epoxide, carboxylic acid, acid chloride, acid halide, acid anhydride, succimidyl ether, aldehyde, ketone, alkyl methane sulfonate, alkyl trifluoromethane sulfonate, alkyl paratoluene methanesulfonate, alkyl halide or an organic multifunctional epoxide. In another embodiment, the polymeric material comprises a reactive chemical group such as a carbodiimide, isocyanate, isothiocyanate, succimidyl ester, epoxide, carboxylic acid, acid chloride, acid halide, acid anhydride, succimidyl ether, aldehyde, ketone, alkyl methane sulfonate, alkyl trifluoromethane sulfonate, alkyl paratoluene methanesulfonate, alkyl halide, amino, sulfhydryl, or an organic multifunctional epoxide. Preferably, the covalent bond between the polymer and the substrate occurs at room temperature, *i.e*. at about twenty to twenty-five degrees Celsius, and occurs spontaneously. Thus, when the composition is applied to a suitable substrate, such as skin, it spontaneously forms one or more covalent bonds with the substrate, leaving an adherent residue conferring antimicrobial activity that is persistent, even after repeated exposure to aqueous solutions.

In another preferred embodiment, the organic polymeric material is chemically reacted with a coupling agent to form an adduct with compounds containing functionalities that are capable of further reacting with and forming covalent chemical bonds with functional groups (e.g. alkyl). ketone, aldehyde, amide, amino, carboxylic acid. hydroxzyl, sulfhydryl, etc.) upon application to a surface comprising one or more of these groups. Such covalent coupling to skin collagen, for example, results in a permanent immobilization of the polymeric antimicrobial material as a film or barrier that can impart immediate antiseptic sanitizing action followed by residual activity that is bactericidal (sanitizing) and/or bacteriostatic (peristence). Suitable coupling agents include compounds with carbodiimide, isocyanate, isothiocyanate, succinimidyl ester, epoxide, carboxylic acid, acid chloride, acid halide, acid anhydride, succimidyl ether, aldehyde, ketone, sulfonyl chloride, alkyl methane sulfonate, alkyl trifluoromethane sulfonate, alkyl paratoluene methane sulfonates and alkyl halide. In a most preferred embodiment, the coupling agent is 1-[3-(Dimethylamino)propyl]-3-ethylcarbodiimide hydrochloride (EDCI).

Thus, in one preferred embodiment, the antimicrobial composition binds non-leachably to a surface by virtue of hydrophobic-hydrophobic interactions. In another embodiment, the antimicrobial composition binds non-leachably to a surface by virtue of electrostatic interactions. In another embodiment, the antimicrobial composition binds non-leachably by virtue of one or more covalent bonds formed between the composition and the surface. These embodiments are not exclusive: the binding of the antimicrobial composition to the surface could be stabilized by any combination of the above elements. In a preferred embodiment, one or more of these interactions occurs rapidly following application of the embodiment a surface. In a particularly preferred embodiment, these interactions can form at approximately room temperature, *i.e*. at about twenty to twenty-five degrees Celsius. In another preferred embodiment, these interactions can form when the composition is applied to skin collagen or to living skin. Preferably, these interactions can form in the presence of an antiseptic selected from the group consisting of ethanol, isopropyl alcohol, chlorhexidine gluconate, iodine, iodine-polyvinylpyrrolidone complex, triclosan, triclorocarban, benzalkonium chloride, and para-chloro-meta-xylenol. More preferably, these interactions can also form in the presence of a thickener, emollient, humectant, skin moisturizing agent or surfactant.

The polymeric antimicrobial material is preferably formulated in a topical composition that includes a second biocidal agent. This second agent comprises any antimicrobial material that' is capable of non-leachably binding to, interacting with or complexing with the polymeric material, but which, when placed in contact with the microorganism, preferentially transfers to the microorganism.

When the dermal antiseptic composition is applied to skin, the polymeric antimicrobial material forms a water-insoluble, non-leachable barrier or film wherein the bioactive functionalities are rendered capable of interacting with, but not diffusing into the bacterial cell membranes of microorganisms contacting it. This phenomenon can be understood by referring to Figure 1, which is a schematic graphic illustration of a preferred embodiment of the present invention in which the organic material is a polymeric biguanide that is rendered substantially water-insoluble by forming a complex with a water insoluble metallic material such as a silver halide, preferably silver iodide. Figure 1A show the polymer barrier film with functionalities capable of bacterial cell wall interactions projecting into the ambient environment, with the silver salt being present both in the complexed form and as entrapped sub-micron particles (as reservoirs). Without wishing to be bound by any theory, it is proposed that when a microorganism contacts the barrier film, the bioactive bisguanidine functionalities disrupt the lipid bilayer constituting the organism's cell membrane, thereby facilitating transfer of silver into the interior of the microorganism or to proteins within the cell membrane. Silver has a greater binding affinity for functionalities in cell membrane proteins than for the polymeric barrier film, and therefore is transferred to the microorganism. The silver is then transported intracellularly wherein it causes protein denaturation and inhibition of DNA synthesis, resulting in cell death. Specifically, it is known that the silver forms complexes with the sulfhydryl and amino groups of the cellular proteins.

In this case, the silver salt is complexed with the self-preserving antimicrobial polymeric material such that the silver is substantially non-leachable into the surrounding environment; neither the silver compound nor silver ions leach from the microbial barrier formed in-situ by the self-preserving polymer even in water contacting environments. The standard Kirby-Bauer zone of inhibition test using test substrates that contain disinfecting composition substantiates this. The absence of a zone in such tests indicates that bioactive components from the composition do not dissolve, elute, leach or provide species in the contacting medium at levels necessary to cause death. Again, not wishing to be bound by theory, it is believed that the silver salt forms complexes with functional groups in the self-preserving antimicrobial polymer, and that the resulting microbial barrier formed on skin containing complexed silver resists leaching into ambient liquids or other materials contacting it (e.g. water, and aqueous solutions including common cleaning liquids).

In a currently preferred embodiment, the polymeric material is poly(hexamethylene-biguanide) hydrochloride

(PHMB.HCl). In another embodiment, the self-preserving antimicrobial polymeric material is rendered water-insoluble by chemically coupling it with a hydrophobic agent such as methylene-bis-N,N-diglycidylaniline (MBDGA) (commercially marketed as Araldite MY-720 by Ciba Giegy). The adduct is made by combining a solution of poly(hexamethylenebiguanide) with a solution of the hydrophobic agent, and reacting the mixture under conditions sufficient to form a PHMB-MBDGA adduct. The ratio ofPHMB to MBDGA preferably is in the range of from about 1:1 to 3:1 by weight. The concentration of the resulting adduct resin preferably is in the range of from about 0.5 to about 20% by weight.

Carriers useful in the present invention include any of generally known creams, lotions, powders, deodorants, sprays, gels, waxes, oils, or ointments, and may include emollients, thickeners, humectants, skin moisturizing agents and surfactants suitable for contact with skin surfaces. In one preferred embodiment, the cream lotion carrier is Soft-Guard protective hand cream lotion (Stahmer Weston Scientific, Portsmouth, NH).

In one embodiment the carrier of the present invention is a dermal antiseptic formulation. Dermal antiseptic formulations suitable for use in the present invention are well-known to those skilled in the art. Such formulations include, but are in no way limited to, formulations comprising alcohols, chlorhexidine gluconate, iodine, iodine-polyvinylpyrrolidone complex, triclosan, triclorocarban and para-chloro-meta-xylenol. Currently preferred antiseptic formulations comprise water-soluble alcohols selected from the group consisting of ethyl alcohol, *n*-propyl alcohol, and isopropyl alcohol. In a preferred embodiment, the alcohol-based skin disinfectant or antiseptic comprises about 30 to about 95%, based on total formulation weight, of a water-soluble alcohol. Suitable dermal antiseptic formulations could comprise, for example, surgical scrubs, pre-operative skin preparations, healthcare personnel handwashes, antiseptic handwashes, antimicrobial soaps, antimicrobial creams, antimicrobial hand sanitizers, antimicrobial deodorants, antimicrobial lotions, antimicrobial gels or other embodiments.

The topical compositions of the present invention are prepared by mixing, dispersing or blending the antimicrobial complexes with the carrier. It is preferable, but not necessary, to thoroughly blend the antimicrobial complex with the carrier to form a homogeneous mixture. The final concentration of the antimicrobial complex in the topical composition may vary depending on the intended use and particular formulation of the ultimate topical composition, as will be appreciated by those of ordinary skill in the art. The final concentration of the organic polycationic material may range from 0.5% to 50% by weight.

The topical antimicrobial compositions of the present invention can also include an optical reporter, e.g., a fluorophore or an optical brightening agent that enables detection of the presence of the topical antimicrobial composition by use of suitable detection devices, such as irradiation by an ultraviolet, fluorescent, infrared, or visible light source. A preferred optical reporter is fluorescent brightener 28 (UVTex-OB, Ciba Specialty Chemicals Corp., Tarrytown, NY). Another preferred optical reporter is Tinopal SFP. When treated skin is examined under light (UV radiation at 365 nm), these optical reporters fluoresce, thereby confirming the presence of the antimicrobial composition. The optical reporter may be blended into the topical antimicrobial composition to a final concentration in the range of 0.05% to 5% by weight, and most preferably around 0.15% by weight.

Methods of the present invention relate to detection of the presence of the antimicrobial composition by first providing the topical antimicrobial composition plus optical reporter as described above, then exposing the surface of interest to a detector capable of detecting the presence of the optical reporter. These methods can be utilized as part of a coherent sanitary program to monitor a subject's compliance with sterile procedures using methods that comprise providing a subject with the topical antimicrobial compositions including optical reporter of the present invention, and subsequently exposing the subject to a detector capable of detecting the presence of the optical reporter on the subject.

Additional methods of the present invention relate to enhancing and extending the duration of efficacy of a dermal antiseptic formulation by adding to the dermal antiseptic formulations described above a polycationic antimicrobial material as described above capable of forming a self-preserving antimicrobial film upon application to skin. The antimicrobial efficacy of the formulation is thus enhanced by the residual antimicrobial activity of the polycationic antimicrobial material.

The invention is further illustrated by the following examples, which are not intended to be limiting in any way. The examples which employ metallic biocides are not within the scope of the invention, but are illustrative of analogous compositions to those within the invention.

<u>Example 1</u>

*Preparation of Surfacine® antimicrobial composition*

Surfacine® antimicrobial composition was prepared according to the methods described in WO-A-98 18330.

The final concentrations of the components in the antimicrobial composition are as follows:

| | |
|---|---|
| PHMB-Araidite MY-720 adduct (2:1 w/w, pH 5.0±0. 1) | 6.7% |

(continued)

| | |
|---|---|
| Silver iodide | 0.8% |
| Potassium iodide | 1.2% |
| Sodium dodecyl sulfate | 3.7% |
| N-methyl-2-pirrolidinone (NMP) | 3.7% |
| Ethanol reagent | 55.1% |
| Water | 14.9% |
| Acetonitrile | 13.8% |

Example 2

*Preparation of Antimicrobial Cream*

SoftGuard protective hand cream lotion (Stahmer Weston Scientific, Portsmouth, NH) was used as the antimicrobial cream formulation base. SoftGuard protective cream is designed to eliminate latex glove irritation. The cream contains the following ingredients: water, sorbitol, 1-hexadecanol, dimethicone, glyceryl monosteatate, lanolin, zinc oxide, sodium lauryl sulfate, methylparaben, propylparaben, quatornium- 15.

Surfacine® antimicrobial composition prepared as described in Example 1 and containing silver iodide, PHMB-Araldite MY-720 adduct and ethanol as the active ingredients was introduced into the cream base to give it antimicrobial properties. Specifically, 600 g of SoftGuard base was blended with 134.5 g of Surfacine antimicrobial formulation into a homogeneous mixture. The resulting antimicrobial cream ("AMC-1") had the following active ingredient content:

| | |
|---|---|
| PHMB-Araldite MY-720 adduct | 1.23% |
| Silver iodide | 0.15% |
| Ethanol reagent | 10.10% |

Example 3

*Preparation of Antimicrobial Cream with Optical Reporter*

Antimicrobial cream as prepared in Example 2 was used and Fluorescent brightener 28 (FB-28) was added as an optical reporter in the antimicrobial cream formulation. Specifically, 0.20 g of FB-28 was mixed with 2 g of glycerol and 2 g of NMP to get a viscous paste. The paste was blended with 40 g of AMC- I formulation, then combined with additional 100 g of AMC-1 and homogenized in a blender.

The resulting antimicrobial cream with optical reporter ("AMC-2") consisted of:

| | |
|---|---|
| AMC-1 (cream base) | 97.08% |
| Glycerol | 1.39% |
| NMP | 1.39% |
| UVTex-OB | 0.14% |

Example 4

*Initial Efficacy of Antimicrobial Hand Cream*

Antimicrobial hand cream samples were tested to determine their efficacy at varied time points. The creams were tested for activity by incubation of the sample with several robust organisms (i.e., *Staphylococcus epidermis-ATCC* #12228, *Escherichia coli* 0157:H7-ATCC #43895, and *Pseudomonas aeruginosa*-ATCC #9027). If a three log or greater decrease in the number of bacteria was detected as compared to the control, the cream was considered to be antimicrobial.

A.) Inoculum Preparation.

A well-isolated colony from a monoculture grown on Tryptic Soy Agar (TSA) was used to inoculate a 10 mL

tube of Tryptic Soy Broth (TSB). The tube was then incubated for 18 hours +/- 2 hours, at 37°C. (The 18 hour culture contained ~ $10^9$ cfu/mL.)

B.) Inoculum Enumeration.

A series of seven 1:10 dilutions in PBS of the 1 X $10^9$ cfu/mL inoculum were performed and the 3 least diluted were discarded. Beginning with the most dilute ($1x10^3$ cfu/mL) and continuing through to the least dilute ($1x10^6$ cfu/mL), 100µL of the solution was added to a plate (each dilution to its own plate). The solutions were evenly spread across the plates according to the spread plate method. The plates were inverted and incubated overnight at 37°C. The plates containing between 30 and 300 distinct colonies were counted. The approximate cfu/mL in the original sample ($1x10^9$) was calculated and recorded.

C.) Sample Preparation.

Cream samples AMC-1 and AMC-2 were diluted at 1:1 with sterile Phosphate Buffered Saline (PBS). The volume of cream was calculated by weight (1 gram of cream = 1 mL of cream). 1 gram of cream was weighed into 50 mL sterile centrifuge tubes. The measured cream was not allowed to adhere to sides of tubes. 1 mL of sterile PBS was added to each tube and the tubes were vortexed to homogenize their contents.

D.) Sample Inoculation and Incubation.

The first sample tube was inoculated with 10 µL of the ~$1x10^9$ cfu/mL organism suspension and vortexed. The inoculated tube was incubated at 22 ± 2°C. At 1 minute intervals, the rest of the samples were inoculated in the same way. At the 10 minute incubation mark, 20 mL of SCP neutralizer broth was added to the first tube and vortexed. The neutralizer was added to the rest of the samples in the order that they were inoculated. The test was repeated with a 30 minute incubation period.

E.) Qualitation.

100µL from each original 50 mL centrifuge tube that has been neutralized was removed to a new 50 mL centrifuge tube with 20 mL neutralizer broth and vortexed thoroughly. The centrifuge tubes were incubated at 37°C for 48 hours and observed for turbidity. The tubes were read as + (growth) or - (no growth) and recorded. To eliminate bacteriostasis as a cause of lack of growth, all subcultures were inoculated with < 100 cfu of organism and re-incubated. Growth of these inocula provided sufficient support to eliminate bacteriostasis as a cause of lack of growth.

F.) Quantitation.

Three (3) serial dilutions at 1:10 in SCP neutralizer broth were performed. Beginning with most dilute and continuing through to the least dilute, 100µL of the solution was added to a plate (each dilution to its own plate). The solutions were evenly spread across the plates according to the spread plate method. 100µL from the original neutralized sample tube was directly plated onto TSA. The plates were inverted and placed in a 37°C incubator. They were incubated for 24 hours or until well-defined colonies were seen. Plates containing between 30 and 300 distinct colonies were counted. The approximate cfu/mL in the sample was calculated and recorded.

G.) Pass Criteria.

A sample passed if the colony counts for the Surfacine antimicrobial cream sample showed greater than or equal to a 3 log decrease with respect to the value of the control cream. Results are shown in Table I below.

TABLE I.

| Initial Efficacy (10 minutes and 30 minutes) | | | | |
|---|---|---|---|---|
| Organism (ATCC#) | Control (Cream Base) Organism, (cfu/mL) | | Antimicrobial Cream (AMC) Organism, (cfu/mL) | |
| | 10 min. | 30 min. | 10 min. | 30 min. |
| *Staphylococcus epidermis (12228)* | $4.4 \times 10^8$ | $4.6 \times 10^8$ | 0 | 0 |

TABLE I. (continued)

| Initial Efficacy (10 minutes and 30 minutes) | | | | |
|---|---|---|---|---|
| Organism (ATCC#) | Control (Cream Base) Organism, (cfu/mL) | | Antimicrobial Cream (AMC) Organism, (cfu/mL) | |
| | 10 min. | 30 min. | 10 min. | 30 min. |
| *Escherichia coli 0157:H7 (43895)* | $7.3 \times 10^7$ | $7.2 \times 10^7$ | 0 | 0 |
| *Pseudomonas aeruginosa (9027)* | $3.1 \times 10^8$ | $2.0 \times 10^8$ | 0 | 0 |

Example 5

*Residual Efficacy of Antimicrobial Hand Cream*

Membrane samples treated with Surfacine® Antimicrobial Hand Cream were tested to determine residual efficacy after application. The membranes were tested for activity by incubation of the sample with several robust organisms (i.e., *Staphylococcus epidermis-ATCC* #12228 and *Escherichia coli* 0157:H7-ATCC #43895). If a three log or greater decrease in the number of bacteria was detected as compared to the control, the coated surface was considered to be active.

A.) Making an 18 hour culture.

A well isolated colony from a monoculture grown on Tryptic Soy Agar (TSA) was taken and used to inoculate a 10 mL tube of Tryptic Soy Broth (TSB). The tube was incubated for 18 hours +/- 2 hours, at 37°C (the 18 hour culture should contain ~ $10^9$ cfu/mL).

B.) Preparation of Inoculum in PBS.

The culture was centrifuged for 15 minutes at 3400 rpm, and the supernatant was removed and discarded. The pellet was resuspended in 10 mL of PBS, and centrifuged for 15 minutes at 3400 rpm. The supernatant was removed and discarded and the pellet was resuspended in 10 mL PBS. The suspension was then centrifuged for 15 minutes at 3400 rpm. The supernatant was removed and discarded and the pellet was resuspended in 10 mL of PBS.

C.) Dilution of the Inoculum in PBS.

A 1:1000 dilution of the $10^9$ cfu/mL solution was performed to get a $10^6$ cfu/mL concentration for inoculation.

D.) Dilution of the Inoculum in Tryptic Soy Broth (TSB).

Three (3) 1:10 dilutions in TSB were performed to get a $10^6$ cfu/mL concentration.

E.) Counting the Inoculum in PBS or TSB.

A series of four 1:10 dilutions in PBS of the 1 X $10^6$ cfu/mL inoculum suspension were carried out as follows:

i) 1:10 $\rightarrow$ 1mL of $1 \times 10^6$ cfu/mL + 9mL PBS = $1 \times 10^5$ cfu/mL suspension.
ii) 1:10 $\rightarrow$ 1mL of $1 \times 10^5$ cfu/mL + 9mL PBS = $1 \times 10^4$ cfu/mL suspension.
iii) 1:10 $\rightarrow$ 1mL of $1 \times 10^4$ cfu/mL + 9mL PBS = $1 \times 10^3$ cfu/mL suspension.
iv) 1:10 $\rightarrow$ 1mL of $1 \times 10^3$ cfu/mL + 9mL PBS = $1 \times 10^2$ cfu/mL suspension.

Beginning with the most dilute (1x10 $1 \times 10^5$ cfu/mL) and continuing through to the least dilute ($1 \times 10^2$ cfu/mL) a plate was inoculated with 100 μL of each dilution. The solution was evenly spread across the plates according to spread plate method, and the plates were incubated overnight at 37°C. Plates containing between 30 and 300 distinct colonies were counted. The approximate cfu/mL in the original sample ($1 \times 10^6$ cfu/mL) was calculated and recorded.

F.) Membrane Sample Preparation.

A 0.2 micron PS membrane was immersed in either control cream or antimicrobial hand cream (AMC-1), and the excess cream was removed. Membranes were dried in a 37°C oven for 1 hour and then cut into 5/8 inch diameter circles and individually placed in wells of 6-well tissue culture plates.

G.) Inoculation of the Membrane Samples.

The $1x10^6$ cfu/mL suspension of organism was used to inoculate each of the membranes with 100 µL of the suspended organism. The well plate containing the samples was then placed in a humidity chamber and incubated at room temperature for 30 and 60 minutes.

H.) Sampling Membranes.

For each membrane, 2 mL of Neutralizer was put in a 50 mL labeled centrifuge tube. Flamed forceps were used to transfer the membrane to the labeled 50 mL tube, ensuring that the membrane was submerged in the Neutralizer. The tubes were then vortexed thoroughly. A 1:10 dilution series in neutralizer was performed as follows:

a) Aliquot 1 mL of liquid from the 50 mL tube after vortexing and add it to 9 mL Neutralizer
b) Vortex to mix.
c) Take 1 ml. of liquid from the 1:10 dilution and add it to 9 mL Neutralizer
d) Vortex to mix.
e) Take 1 mL of liquid from the second 1:10 dilution and add to 9 mL Neutralizer.
f) Vortex to mix.

A TSA plate was labeled for each sample dilution. 100 µL was pipetted onto a separate plate for each dilution tube in a series. Starting with the most dilute, the sample was spread carefully over the agar leaving an even film. A fresh sterile plate spreader was used for each dilution series. The plates were inverted and placed in a 37°C incubator for 24 hours or until well defined colonies were seen.

I.) Counting Plates.

Plates were removed from the incubator after the allotted time. The number of colonies on plates containing between 30 and 300 colonies were counted. The approximate cfu/mL in the original sample was calculated and recorded.

J.) Results.

A sample passed when the count for the sample showed greater than or equal to a 3 log reduction from the value of the control. Results are shown in Table II below.

TABLE II.

| Residual Efficacy (1 hr. at 37°C) after application | | | | |
|---|---|---|---|---|
| Organism (ATCC#) | Control (Cream Base) Organism, (cfu/mL) | | Antimicrobial Cream (AMC) Organism, (cfu/mL) | |
| | 30 min. | 60 min. | 30 min. | 60 min. |
| *Escherichia coli 0157:H7 (43895)* | $4.5 \times 10^5$ | $3.1 \times 10^5$ | 0 | 0 |
| *Pseudomonas aeruginosa (9027)* | $8.1 \times 10^4$ | $2.0 \times 10^5$ | 0 | 0 |

Example 6:

*Alcohol-containing antiseptic hand sanitizer formulation containing self-preserving polymer preservative*

The formulation was prepared with Cosmocil CQ™ (PHMB = 20 wt. %) in ethyl alcohol.
Formulation:

Cosmocil CQ™ = 0.5 - 10.0%

Alcohol (denatured, Ethanol 94-96%, Isopropanol = 4-6%) = 70 - 80%

Water =10 - 29.5%

A typical formulation is:

Cosmocil CQ™ = 2.5%

Alcohol (denatured, Ethanol 94-96%, Isopropanol = 4-6%) = 70%

Water = 27.5%

Required amount of water was introduced into alcohol followed by the Cosmocil CQ™ and stirred for 10 minutes. The solution was clear and colorless.

Example 7:

*Alcohol-containing antiseptic formulation containing self-preserving polymer preservative with a skin moisturizing thickener*

The formulation was prepared with Cosmocil CQ™ in alcohol with Celquat™ SC-230M thickener.
Formulation:

Cosmocil CQ™ = 0.5 - 10%

Alcohol = 70 - 80%

Celquat™ - Thickener = 0.2 - 5%

Water = 5 - 29.3%

A typical formulation is:

Cosmocil CQ™ = 0.5%

Alcohol = 70%

Celquat™ - Thickener = 1%

Water = 28.5%

Required amount of the Celquat™ SC-230 M was sprinkled into water slowly and was allowed to stir overnight at 50° C. Required amount of alcohol was introduced into the water with thickener with stirring, followed by Cosmocil CQ™ in drops. The entire mixture was stirred for 1 more hour. The thickened hand sanitizer is clear and colorless.

Example 8:

*Alcohol-containing antiseptic formulation containing self-preserving polymer complexed with silver compound*

The formulation was prepared with Cosmocil CQ™ & Ag in alcohol with Celquat™ SC-230M thickener. Formulation:

Cosmocil CQ™ =0.5 - 10%

AgI = 0.005 - 0.5%

Alcohol = 70 - 80%

Celquat™ - Thickener =0.5 - 5%

Water =4 - 23.0%

Other additives = 0.5 - 6%

A typical formulation consists of:

Cosmocil CQ™ = 2.5%

AgI = 0.05%

Alcohol = 72.6%

Celquat™ - Thickener = 1%

Water = 23.0%

KI = 0.20%

Sodium dodecyl sulfate (SDS) = 0.30%

N-methyl pyrrolidinone (NMP)= 0.35%

Required amount of the Celquat™ SC-230 M was sprinkled into water slowly and was allowed to stir overnight at 50°C. Required amount of alcohol was introduced into the water with thickener with stirring. To the solution containing Cosmocil™, SDS and NMP, the required amount of AgI and KI was introduced and stirred well to get a homogenous mixture. The AgI containing mixture was introduced into the Celquat™ containing aqueous alcohol mixture in drops. The entire mixture was stirred for 1 more hour. The thickened hand sanitizer looks clear and slightly yellow in color.

Example 9:

*Iodine-Povidone dermal antiseptic containing antiseptic formulation containing self-preserving polymer complex*

The formulation was prepared involving Betadine™, Cosmocil CQ™ (PHMB = 20 wt. %) and Ag. The main ingredient of Betadine™ is Povidone-Iodine, 10 % which is the equivalent of 1% available iodine. Povidone is a 1-Ethenyl-2-pyrrolidinone homopolymer compounded with iodine; polyvinylpyrrolidone-iodine complex. Other inactive ingredients are citric acid, dibasic sodium phosphate, glycerin and others.
Formulation:

Cosmocil CQ™ = 0.5 - 10.0%

AgI = 0.005 - 0.5%

1. Alcohol (denatured, Ethanol 94-96%, Isopropanol = 4-6%) = 10 - 20%

Betadine™ =70 - 90%

Other additives = 0.5 - 6%

Required amount of alcohol was introduced into the Betadine™ solution and mixed well. To the solution containing Cosmocil™, SDS and NMP, the required amount of AgI and KI was introduced and stirred well to get a homogenous mixture. The AgI containing mixture was introduced into the alcohol solution of Betadine™ in drops and mixed well for a period of 30 minutes.

Example 10:

*Chlorhexidine dermal antiseptic containing antiseptic formulations containing self-preserving polymer complex*

The formulations were prepared using (i) Hibiclens™ and (ii), PHMB-MBDGA adduct complexed with silver. The active ingredient of Hibiciens™ is 4% w/v Hibitane™ (Chlorhexidine gluconate). The active ingredients in Hibistat are 0.5% Chlorhexidine gluconate and 70% isopropanol.
Formulation:

PHMB-MBDGA adduct = 0.5 - 10.0%

Silver compound = 0.005 - 0.5%

Hibiclens™ = 80 - 95%

Required amount of alcohol was introduced into the biguanide-epoxide resin and to the alcoholic solution

containing modified biguanide, sodium dodecyl sulfonate (SDS) and N-methyl-2-pyrrolidone (NMP). Silver nitrate and KI were subsequently introduced and stirred well to get a homogenous mixture. The resulting antimicrobial polymer silver complex solution was introduced dropwise into Hibiclens™ and stirred for a period of 30 minutes. to form a completely miscible solution.

Example 11:

*Neutralization of Collagen for in-vitro residual efficacy testing of hand sanitizer formulations*

Materials:

Collagen sheets

| | |
|---|---|
| Phosphate Buffer pH 6.8 | 2 liters |

Procedure:

Collagen sheets were placed in a. 11×13" glass coming dishes and clamped at each comer to prevent the ends from curling up. Preheated phosphate buffer containing $NaBH_4$ was carefully poured over each collagen sheet and gently shaken to saturate the entire sheet. The dishes were placed in a 50°C oven for 10-15 minutes. After reduction, the samples were rinsed with 40-50°C DI $H_2O$ for ~3-5 minutes (static). The above procedure was repeated three times,
after which they were hung to dry and packaged appropriately.

Example 12:

*Residual Efficacy of Hand Sanitizer formulation on Collagen*

The following study was undertaken to characterize the residual antimicrobial efficacy of a Surfacine Hand Sanitizer formulation after product application and subsequent water contact.

Sample Preparation:

Collagen sheets were neutralized according to Example 11. The sheets were cut into squares with length greater than the internal diameter of an open ended x-ray cup with compression rings. Collagen squares were attached to the open ended x-ray cups with compression rings. The formulations were applied on the side with topography consistent with human skin.

Product Application:

The test formulation was applied to the collagen rings with a pipet and spread over the surface to obtain a ratio of (volume of product) to (surface area of collagen) of ~10µL/cm$^2$ (approximately 70µL per prepared collagen sample cup). The sample was allowed to dry.

Environmental Stress:

To characterize the efficacy of the formulation after a simulated water rinse, collagen rings were contacted with de-ionized water at 40°C maintained at a flow rate to ~1L/minute. for 30 seconds, after which they were air dried.

Inoculation:

An overnight culture of *Pseudomonas aeruginosa* ATCC # 9027 grown in tryptic soy broth (TSB). The culture was adjusted to contain ~10$^8$ cfu/ml in TSB and 10µL was inoculated onto the center of a collagen sampling ring and was spread with a pipet tip. Samples were incubated at 22 ± 2°C for 1 hour in a humidity chamber maintained at >90% relative humidity. The collagen samples were excised from the rings with a sterile scalpel and transferred with sterile

forceps into 10ml of sterile Dey-Engley neutralizing broth (D/E broth) and vortexed. Quantitative assays were performed by preparing 1:10 serial dilutions in phosphate-buffered saline to extinction and 100µL aliquots from the dilution series were transferred and plated by a standard spreading method. Plates were inverted and incubated for 24-48 hours at 37°C or until colonies were visible. Qualitative assays were performed by incubating recovered collagen in 10ml D/E broth for 24-48 hours at 37°C and observing for turbidity.

Persistent antimicrobial activity is characterized in this experiment by a two log reduction in organism viability when compared to the control. As shown in Table 3, addition of a PHMB-MBDGA adduct, formulated with a silver compound, to alcohol-based formulations significantly increases the imparted residual antimicrobial activity of the formulation. This activity persists even after rinsing the surface with water. As shown in Table 4, a PHMB-MBDGA adduct formulated with a silver compound also enhances the residual antimicrobial activity of surgical scrubs, even after subsequent rinsing of the surface. In each case, the PHMB-MBDGA adduct improved the rinsed residual activity by at least two orders of magnitude.

**TABLE III. Residual Antimicrobial Efficacy of Hand Sanitizer on Neutralized Collagen After Water Contact**
**Comparison of Alcohol Based Formulations with and without Surfacine**

| | | | | | | |
|---|---|---|---|---|---|---|
| Purell (62% EtOH) | 0.5 | N/T | | N/T | N/T | |
| 70% EtOH | 0.2 | N/T | | N/T | N/T | |
| 70% EtOH w/0.5% Cosmocil CQ | N/T | N/T | | 1.5 | N/T | |
| [illegible] | | | | | | |
| 62% EtOH, 0.25% Cosmocil CQ-MBDGA w/ silver | 1.3 | N/T | | N/T | N/T | |
| 70% EtOH, 0.5% Cosmocil CQ-MBDGA w/ silver | 2.2 | N/T | | >1.9 | N/T | |
| 70% EtOH, 0.5% Cosmocil CQ-MBDGA w/ silver, no thk. | 4.0 | 4.5 | | >1.9 | 2.5 | |

N/T: Not Tested

**TABLE IV. Residual Antimicrobial Efficacy of Hand Sanitizer on Neutralized Collagen After Water Contact**
**Comparison of Conventional Scrub Products with and without Surfacine**

| | | | | | | |
|---|---|---|---|---|---|---|
| Hibistat (70% isopr. alc., 0.5% chlorhexidine gluc. w/o thickener) | 5.0 | 4.5 | | N/T | -0.1 | |
| Betadine (10% Povidone-Iodine) | N/T | N/T | | N/T | 1.3 | |
| Hibiclens (4.0% chlorhexidine gluconate) | N/T | N/T | | N/T | 2,9 | |
| [illegible] | | | | | | |
| Hibistat + 0.5% Cosmocil CQ-MBDGA adduct w/ silver | N/T | N/T | | N/T | 2.8 | |
| Betadine + 0.5% Cosmocil CQ-MBDGA adduct w/ silver | N/T | N/T | | N/T | 2.4 | |
| Hibiclens + 0.5% Cosmocil CQ-MBDGA adduct w/ silver | N/T | N/T | | N/T | 4.9 | |

N/T: Not Tested

<u>Example 13:</u>

*Residual efficacy (persistence) provided by Self-Preserving Antimicrobial Polymer (SPAP) in alcohol containing dermal antiseptics*

This study was carried out to establish that the *in vitro* antimicrobial studies with collagen sheets accurately reproduce an *in vivo* phenomenon. The experiments were carried out as described for Example 12, except that pigskin or a region of forearm was used in place of the collagen rings where indicated. As shown in Table 5, the residual efficacy of a dermal antiseptic as measured on a region of forearm correlates well with experiments done on collagen rings, and does not correlate as well with experiments done on pigskin.

In Table 5, recovery of control organisms to within 1/2 log is reported as "+++", whereas in an experiment in

which only 1-10% of control organisms were recovered is reported as a control organism recovery of "+".

**TABLE V.** Correlation of *in vitro* substrate to *in vivo* studies.

| Study | | Vivo | Vitro | |
|---|---|---|---|---|
| Substrate | | Forearm | Pigskin | Collagen |
| Incubation Temperature (in degrees Celsius) | | ~30 | ~30 | RT |
| Control Organism Recovery | | + | +++ | +++ |
| **Sample** | **Description** | **Residual** | | |
| Control | No treatment T=0 | 5.9 | | 6.2 |
| Control | No treatment T=60m | 4.7 | 4.9 | 6.0 |
| Hibistat™ | 70% isopropyl alcohol, 0.5% chlorhexidine gluconate, no thickener | 1.3 | 4.4 | 1.0 |
| 5002-74-2 | 70%EtOH, 0.5% Cosmocil CQ™-MBDGA | 3.2 | 3.6 | 3.8 |
| 5002-75-2 | 70%EtOH,0.5% Cosmocil CQ™-MBDGA, no thickener | ~2.0 | 4.3 | 2.0 |

Average $\log_{10}$ cfu/sample reported.

Example 14:

*Demonstration of efficacy of instant invention when used in surgical scrub*

The ability of Cosmocil CQ™ or Surfacine® to provide persistent antimicrobial activity was determined using the "Standard Test Method for Evaluation of Surgical Hand Scrub Formulations" described in protocol E 1115-91 of the Annual Book of ASTM Standards, Vol. 11.05, published in September, 1991 by the American Public Health Association, Inc. of Washington, DC. Participants in the study washed their hands and forearms with the surgical scrub formulation once on days 1 and 5 and three times per day on days 2, 3, and 4. The number of elutable bacteria was determined following the only scrub of days 1 and 5 and following the first scrub of day 2. The number of elutable bacteria was determined both immediately following the scrub and six hours thereafter. At least four subjects were tested at each time point.

As shown in Figure 2, subjects who used a surgical scrub containing Cosmocil CQ™ demonstrated significant antimicrobial activity that persisted even six hours following use of the scrub. The antimicrobial activity was most pronounced after five days of use of the scrub, consistent with the persistent nature of the antimicrobial activity. As shown in Figure 3, subjects who used a surgical scrub containing Surfacine®, formulated as described in Example 1, also demonstrated persistent antimicrobial activity. This activity could be detected for a composition comprising 0.2% Surfacine®, but was more apparent for a composition comprising 0.35% Surfacine® and even more apparent for a composition comprising 0.5% Surfacine®.

**Claims**

1. Use of an antimicrobial composition comprising a water-insoluble adduct of a polycationic organic polymer with a hydrophobic agent, wherein the water-insoluble adduct is free of any additional biocidal material that is capable of being non-leachably bound to the adduct.

2. Use according to claim 1 wherein the water-insoluble adduct is free of other antimicrobial compounds.

3. Use according to claim 1 or claim 2 wherien the polycationic organic polymer is a polycationic biguanide polymer and the hydrophobic agent is a hydrophobic crosslinking agent.

4. Use according to any of Claims 1 to 3, wherein the antimicrobial composition is a dermal antimicrobial composition.

5. Use according to any of claims 1 to 3, wherein the antimicrobial composition is a surgical scrub.

**6.** A dermal antimicrobial composition without an antimicrobial metal, the dermal antimicrobial composition comprising:

a water-insoluble adduct of a hydrophobic polycationic organic polymer with a hydrophobic agent; and a marker.

**7.** A dermal antimicrobial composition according to Claim 6; wherein the marker is an optical reporter.

**8.** A dermal antimicrobial composition according to Claim 6, wherein the marker comprises a compound detectable under ultraviolet, visible, or infrared irradiation.

**9.** A dermal antimicrobial composition according to Claim 8, wherein the marker comprises a compound that fluoresces under ultraviolet or infrared light.

**10.** A dermal antimicrobial composition according to Claim 9, wherein the marker is selected from the group consisting of Fluorescent Brightener-28 and Tinopal SFP.

**11.** A use or dermal antimicrobial composition according to any one of Claims 2 to 10, wherein the dermal antimicrobial composition comprises a surgical scrub, a pre-operative skin preparation, a healthcare personnel handwash, an antiseptic handwash, antimicrobial soap, antimicrobial cream, antimicrobial hand sanitizer, antimicrobial deodorant, antimicrobial gel, antimicrobial lotion, antimicrobial spray, antimicrobial foam, antimicrobial powder, antimicrobial wax, antimicrobial oil or antimicrobial ointment.

**12.** A use or dermal antimicrobial composition according to any one of Claims 2 to 11, wherein the dermal antiseptic composition further comprises 30% to 98% by weight of at least one alcohol selected from the group consisting of ethyl alcohol, n-propanol, and isopropanol.

**13.** A use or dermal antimicrobial composition according to any one of Claims 2 to 11, wherein the dermal antiseptic composition further comprises an antiseptic selected from the group consisting of ethanol, isopropyl alcohol, chlorhexidine gluconate, iodine, iodine-polyvinylpyrrolidone complex, triclosan, triclorocarban, benzalkonium chloride and para-chloro-meta-xylenol:

**14.** A use or dermal antimicrobial composition according to any one of Claims 2 to 13, wherein the dermal antiseptic composition further comprises a thickener, emollient, humectant, skin moisturizing agent or. surfactant.

**15.** A use or dermal antimicrobial composition according to any one of Claims 2 to 14, wherein the hydrophobic polycationic organic polymer comprises biguanide moieties.

**16.** A method for detecting the presence of an antimicrobial composition on a surface, the method comprising the steps of:

(i) providing on the surface the dermal antimicrobial composition of claims 6; and
(ii) exposing the surface to a detector capable of detecting. the presence of the marker of Claim 6 on the surface.

**17.** A method for monitoring a subject's compliance with aseptic procedures, the method comprising the steps of:

(i) providing to the subject the dermal antimicrobial composition of Claim 6; and
(ii) subsequently exposing the subject to a detector capable of detecting the presence of the marker of Claim 6 on the subject.

**18.** Use of a polycationic organic polymer in a dermal antimicrobial. composition, the polymer comprising a reactive group that forms a covalent bond with skin upon application at room temperature.

**Patentansprüche**

**1.** Verwendung einer antimikrobiellen Zusammensetzung, umfassend ein in Wasser unlösliches Addukt eines polykationischen organischen Polymers mit einem hydrophoben Mittel, wobei das in Wasser unlösliche Addukt frei

von zusätzlichem Biozidmaterial ist, das nicht auslaugbar an das Addukt gebunden werden kann.

**2.** Verwendung nach Anspruch 1, wobei das in Wasser unlösliche Addukt frei von anderen antimikrobiellen Verbindungen ist.

**3.** Verwendung nach Anspruch 1 oder Anspruch 2, wobei das polykationische organische Polymer ein polykationisches Biguanidpolymer und das hydrophobe Mittel ein hydrophobes Vernetzungsmittel ist.

**4.** Verwendung nach einem der Ansprüche 1 bis 3, wobei die antimikrobielle Zusammensetzung eine dermale antimikrobielle Zusammensetzung ist.

**5.** Verwendung nach einem der Ansprüche 1 bis 3, wobei die antimikrobielle Zusammensetzung ein chirurgisches Scheuermittel ist.

**6.** Dermale antimikrobielle Zusammensetzung ohne antimikrobielles Metall, wobei die dermale antimikrobielle Zusammensetzung Folgendes umfasst:

ein in Wasser unlösliches Addukt eines hydrophoben polykationischen organischen Polymers mit einem hydrophoben Mittel; und einen Marker.

**7.** Dermale antimikrobielle Zusammensetzung nach Anspruch 6, wobei der Marker ein optischer Reporter ist.

**8.** Dermale antimikrobielle Zusammensetzung nach Anspruch 6, wobei der Marker eine Verbindung umfasst, die unter ultravioletten, sichtbaren oder infraroten Strahlen erfassbar ist.

**9.** Dermale antimikrobielle Zusammensetzung nach Anspruch 8, wobei der Marker eine Verbindung umfasst, die unter ultraviolettem oder infrarotem Licht fluoresziert.

**10.** Dermale antimikrobielle Zusammensetzung nach Anspruch 9, wobei der Marker ausgewählt ist aus der Gruppe bestehend aus Fluorescent Brightener 28 und Tinopal SFP.

**11.** Verwendung oder dermale antimikrobielle Zusammensetzung nach einem der Ansprüche 2 bis 10, wobei die dermale antimikrobielle Zusammensetzung Folgendes umfasst: ein chirurgisches Scheuermittel, ein präoperatives Hautpräparat, ein Handreinigungsmittel für medizinisches Personal, ein antiseptisches Handreinigungsmittel, eine antimikrobielle Seife, eine antimikrobielle Creme, ein antimikrobielles Handdesinfektionsmittel, ein antimikrobielles Deodorant, ein antimikrobielles Gel, eine antimikrobielle Lotion, ein antimikrobielles Spray, einen antimikrobiellen Schaum, ein antimikrobielles Puder, ein antimikrobielles Wachs, ein antimikrobielles Öl oder eine antimikrobielle Salbe.

**12.** Verwendung oder dermale antimikrobielle Zusammensetzung nach einem der Ansprüche 2 bis 11, wobei die dermale antiseptische Zusammensetzung ferner 30 bis 98 Gew.-% von wenigstens einem Alkohol umfasst, ausgewählt aus der Gruppe bestehend aus Ethylalkohol, n-Propanol und Isopropanol.

**13.** Verwendung oder dermale antimikrobielle Zusammensetzung nach einem der Ansprüche 2 bis 11, wobei die dermale antiseptische Zusammensetzung ferner ein Antiseptikum umfasst, ausgewählt aus der Gruppe bestehend aus Ethanol, Isopropylalkohol, Chlorhexidingluconat, Iod, Iod-Polyvinylpyrrolidon-Komplex, Triclosan, Triclorocarban, Benzalkoniumchlorid und para-Chloro-meta-Xylenol.

**14.** Verwendung oder dermale antimikrobielle Zusammensetzung nach einem der Ansprüche 2 bis 13, wobei die dermale antiseptische Zusammensetzung ferner ein Verdickungsmittel, ein Erweichungsmittel, ein Feuchthaltemittel, ein Hautbefeuchtungsmittel oder ein Tensid umfasst.

**15.** Verwendung oder dermale antimikrobielle Zusammensetzung nach einem der Ansprüche 2 bis 14, wobei das hydrophobe polykationische organische Polymer Biguanidanteile umfasst.

**16.** Verfahren zum Erfassen der Anwesenheit einer antimikrobiellen Zusammensetzung auf einer Oberfläche, umfassend die folgenden Schritte:

(i) Aufbringen der dermalen antimikrobiellen Zusammensetzung nach Anspruch 6 auf die Oberfläche; und
(ii) Inkontaktbringen der Oberfläche mit einem Detektor, der die Anwesenheit des Markers nach Anspruch 6 auf der Oberfläche erfassen kann.

**17.** Verfahren zum Überwachen der Einhaltung von Desinfektionsverfahren durch eine Person, umfassend die folgenden Schritte:

(i) Aufbringen der dermalen antimikrobiellen Zusammensetzung nach Anspruch 6 auf die Person; und
(ii) nachfolgend Inkontaktbringen der Person mit einem Detektor, der die Anwesenheit des Markers nach Anspruch 6 auf der Person erfassen kann.

**18.** Verwendung eines polykationischen organischen Polymers in einer dermalen antimikrobiellen Zusammensetzung, wobei das Polymer eine reaktive Gruppe umfasst, die eine kovalente Bindung mit der Haut nach einer Applikation bei Raumtemperatur bildet.

**Revendications**

**1.** Utilisation d'une composition antimicrobienne comprenant l'adduit insoluble dans l'eau d'un polymère organique polycationique et d'un agent hydrophobe, ledit adduit insoluble dans l'eau étant exempt de tout matériel biocide additionnel pouvant être lié à l'adduit d'une manière non lessivable.

**2.** Utilisation selon la revendication 1, dans laquelle l'adduit insoluble dans l'eau est exempt d'autres composés antimicrobiens.

**3.** Utilisation selon la revendication 1 ou la revendication 2, dans laquelle le polymère organique polycationique est un polymère polycationique de biguanide et l'agent hydrophobe est un agent de réticulation hydrophobe.

**4.** Utilisation selon l'une quelconque des revendications 1 à 3, dans laquelle la composition antimicrobienne est une composition antimicrobienne à usage cutané.

**5.** Utilisation selon l'une quelconque des revendications 1 à 3, dans laquelle la composition antimicrobienne est une solution de lavage chirurgical des mains.

**6.** Composition antimicrobienne à usage cutané ne contenant pas de métal à action antimicrobienne, la composition antimicrobienne à usage cutané comprenant :

l'adduit insoluble dans l'eau d'un polymère organique polycationique hydrophobe et d'un agent hydrophobe ; et un marqueur.

**7.** Composition antimicrobienne à usage cutané selon la revendication 6, dans laquelle le marqueur est un traceur optique.

**8.** Composition antimicrobienne à usage cutané selon la revendication 6, dans laquelle le marqueur contient un composé décelable dans l'ultraviolet, dans le visible et dans l'infrarouge.

**9.** Composition antimicrobienne à usage cutané selon la revendication 8, dans laquelle le marqueur contient un composé qui est fluorescent en lumière ultraviolette ou infrarouge.

**10.** Composition antimicrobienne à usage cutané selon la revendication 9, dans laquelle le marqueur est sélectionné parmi le groupe consistant en l'agent azurant fluorescent 28 et le Tinopal SFP.

**11.** Utilisation d'une composition antimicrobienne à usage cutané selon l'une quelconque des revendications 2 à 10, ladite composition antimicrobienne à usage cutané se présentant sous la forme d'une solution de lavage chirurgical, de préparation cutanée pré-opératoire, de lavage des mains par le personnel soignant ou de lavage antiseptique des mains, d'un savon antimicrobien, d'une crème antimicrobienne, d'un produit nettoyant antimicrobien des mains, d'un déodorant antimicrobien, d'un gel antimicrobien, d'une lotion antimicrobienne, d'un aérosol antimicrobien, d'une mousse antimicrobienne, d'une poudre antimicrobienne, d'une cire antimicrobienne, d'une huile

antimicrobienne ou d'une pommade antimicrobienne.

**12.** Utilisation d'une composition antimicrobienne à usage cutané selon l'une quelconque des revendications 2 à 11, ladite composition antimicrobienne à usage cutané contenant également de 30 % à 98 % en poids d'au moins un alcool sélectionné parmi le groupe consistant en l'alcool éthylique, le n-propanol et l'isopropanol.

**13.** Utilisation d'une composition antimicrobienne à usage cutané selon l'une quelconque des revendications 2 à 11, ladite composition antimicrobienne à usage cutané contenant également un antiseptique sélectionné parmi le groupe consistant en l'éthanol, l'alcool isopropylique, le gluconate de chlorhexidine, l'iode, le complexe poyvinylpyrrolidone-iode, le triclosan, le trichlorocarban, le chlorure de benzalkonium et le parachlorométaxylénol.

**14.** Utilisation d'une composition antimicrobienne à usage cutané selon l'une quelconque des revendications 2 à 13, ladite composition antimicrobienne à usage cutané contenant également un épaississant, un émollient, un humectant, un hydratant de la peau ou un agent tensio-actif.

**15.** Utilisation d'une composition antimicrobienne à usage cutané selon l'une quelconque des revendications 2 à 14, dans laquelle le polymère organique polycationique hydrophone contient des fragments biguanides.

**16.** Méthode de détection de la présence d'une composition antimicrobienne sur une surface, qui comprend les étapes suivantes :

(i) le revêtement de ladite surface avec la composition antimicrobienne à usage cutané selon la revendication 6 ; et

(ii) l'exposition de ladite surface à un détecteur capable de mettre en évidence la présence du marqueur de la revendication 6 à la surface.

**17.** Méthode de monitorage de l'adhésion d'un sujet aux procédures aseptiques, la méthode comprenant les étapes suivantes :

(i) la mise à la disposition au sujet de la composition antimicrobienne à usage cutané de la revendication 6 ; et
(ii) l'exposition ultérieure du sujet à un détecteur capable de mettre en évidence la présence du marqueur de la revendication 6 sur le sujet.

**18.** Utilisation d'un polymère organique polycationique dans une composition antimicrobienne à usage cutané, ledit polymère comprenant un groupement réactif qui forme une liaison covalente avec la peau durant l'application à température ambiante.

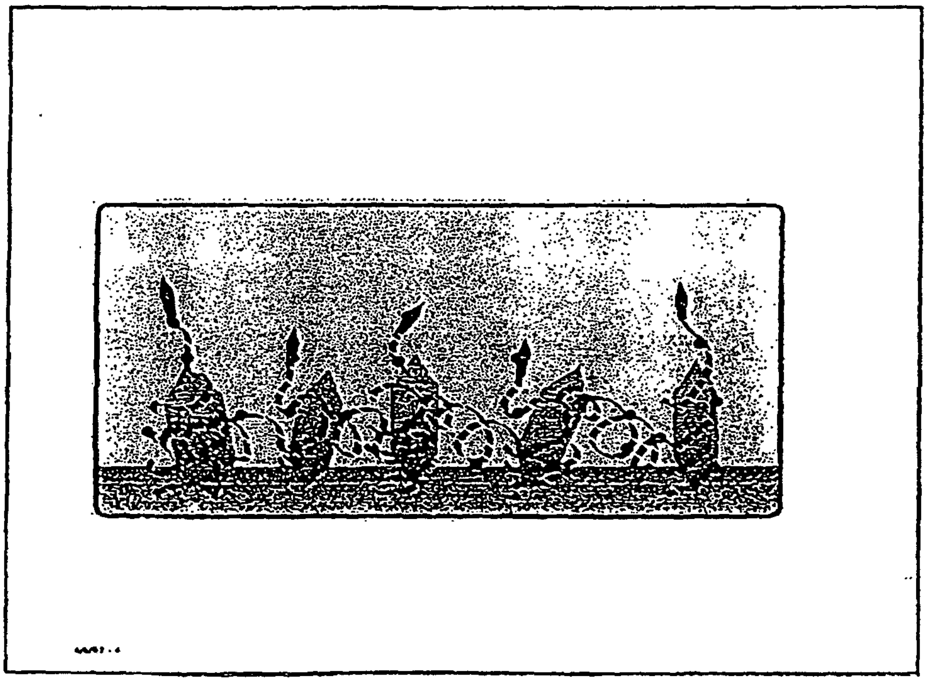

FIG 1A

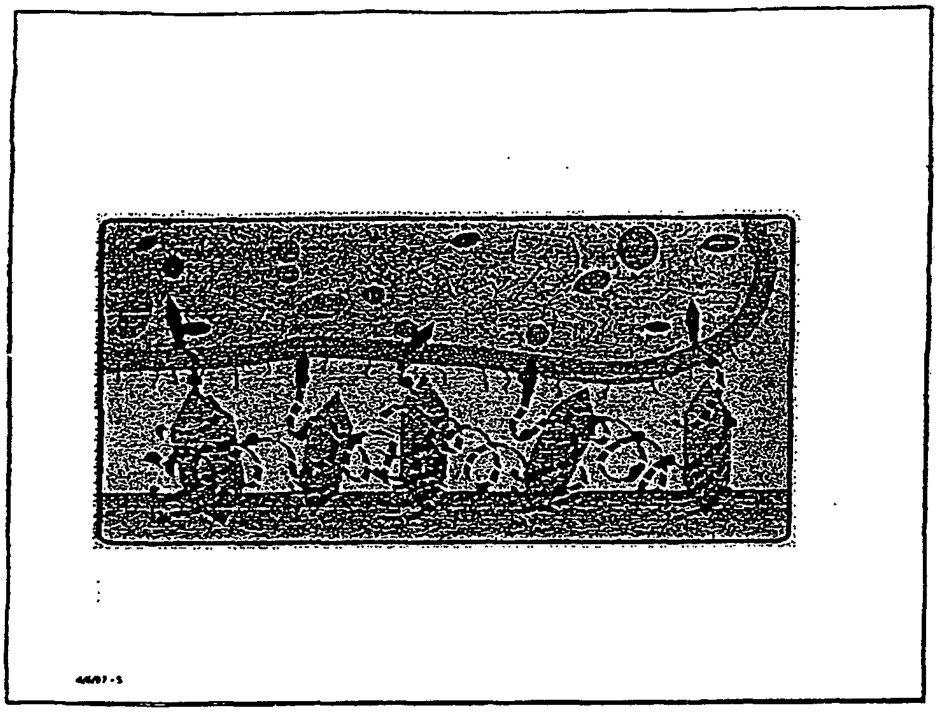

FIG 1B

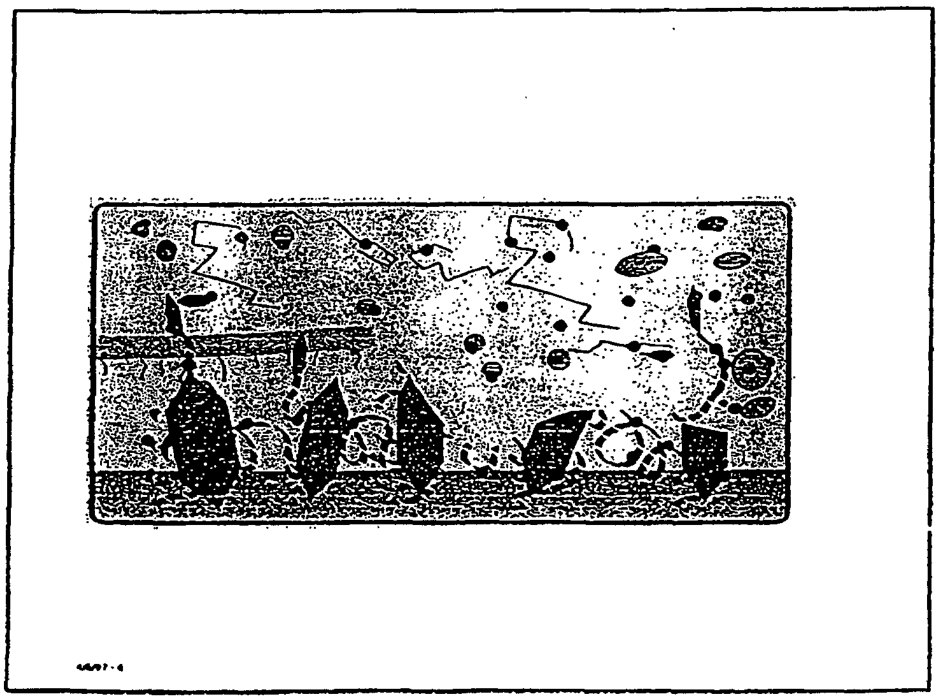

FIG 1C

Figure 2.

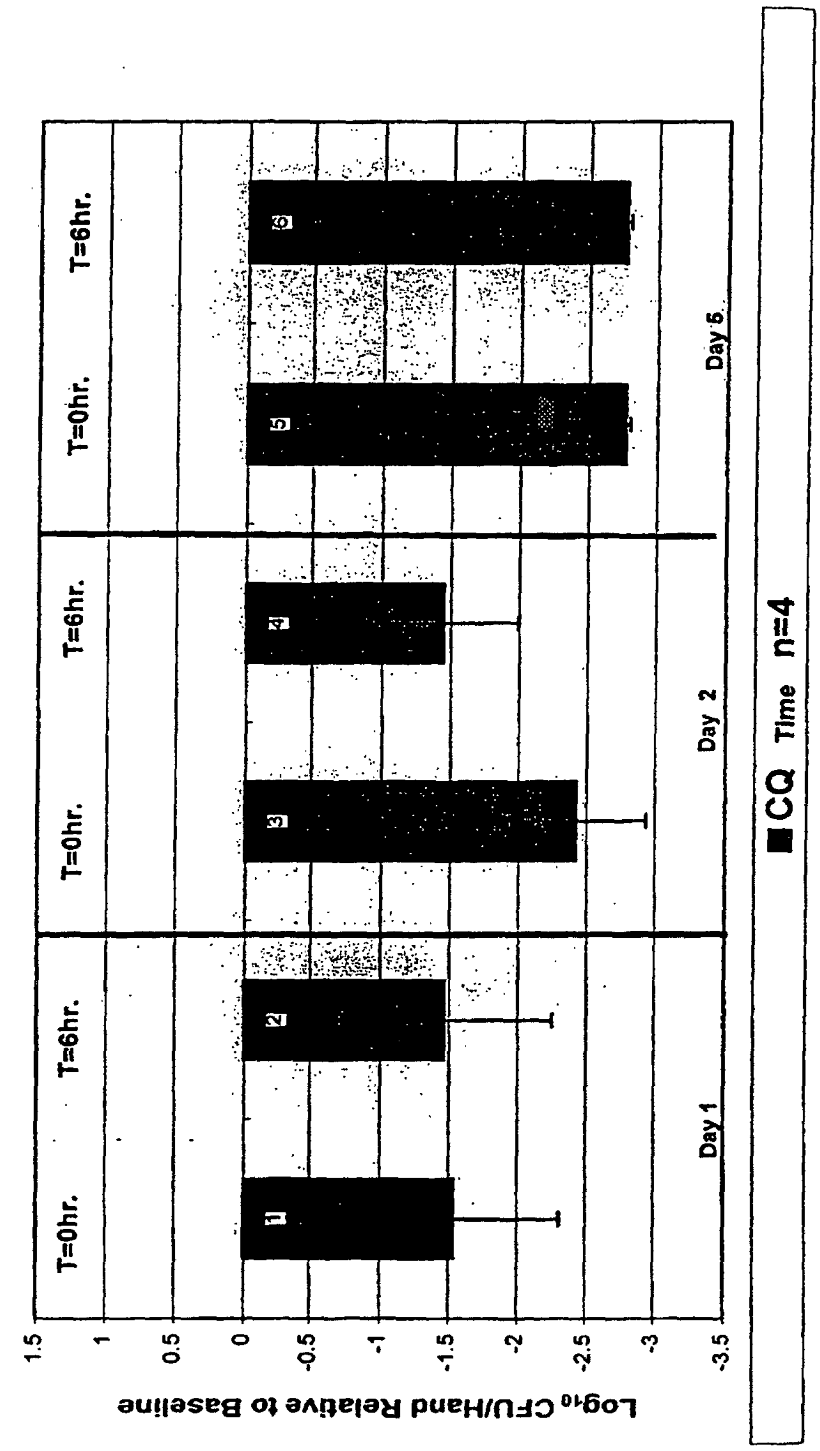
Log10 CFU/Hand Relative to Baseline
1.5
1
0.5
0
-0.5
-1
-1.5
-2
-2.5
-3
-3.5
T=0hr.
T=6hr.
T=0hr.
T=6hr.
T=0hr.
T=6hr.
1
2
3
4
5
6
Day 1
Day 2
Day 5
CQ Time n=4

Figure 3.

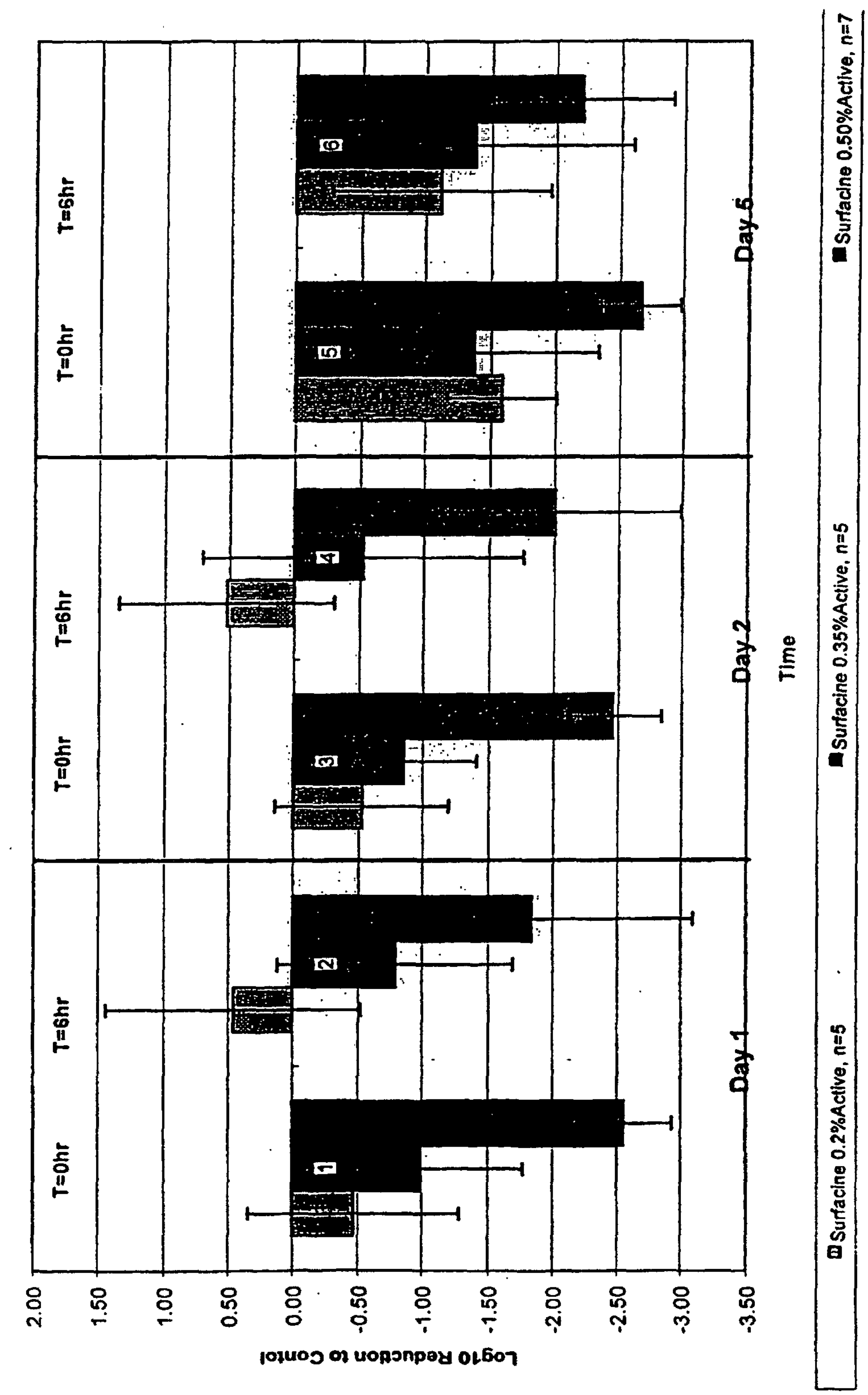
T=0hr
T=6hr
T=0hr
T=6hr
T=0hr
T=6hr
1
2
3
4
5
6
Day 1
Day 2
Day 5
2.00
1.50
1.00
0.50
0.00
-0.50
-1.00
-1.50
-2.00
-2.50
-3.00
-3.50
Log10 Reduction to Contol
Time
Surfacine 0.2%Active, n=5
Surfacine 0.35%Active, n=5
Surfacine 0.50%Active, n=7